# EUROPEAN PATENT APPLICATION

(11) **EP 4 779 016 A1**
(43) Date of publication of application: **22.07.2026**
(21) Application number: 23951728.7
(22) Date of filing: 11.09.2023
(51) Int. Cl.: C12N 5/073, C12N 5/0735

(54) **EMBRYO-LIKE MODEL, AND CONSTRUCTION METHOD THEREFOR AND USE THEREOF**

(71) Applicant: BGI Research Shenzhen, Yantian District Shenzhen, Guangdong 518083 (CN); Guangzhou Institutes of Biomedicine and Health, Chinese Academy of Sciences, Guangzhou, Guangdong 510530 (CN)
(72) Inventor: ESTEBAN, Miguel, Shenzhen, Guangdong 518083 (CN); LAI, Yiwei, Shenzhen, Guangdong 518083 (CN); MAZID, Md·Abdul, Shenzhen, Guangdong 518083 (CN); LI, Jinxiu, Shenzhen, Guangdong 518083 (CN); JIA, Wenqi, Shenzhen, Guangdong 518083 (CN); FU, Lixin, Shenzhen, Guangdong 518083 (CN); ZUO, Jing, Shenzhen, Guangdong 518083 (CN); LI, Wenjuan, Shenzhen, Guangdong 518083 (CN); WU, Liang, Shenzhen, Guangdong 518083 (CN)
(74) Representative: Potter Clarkson
(86) International application number: PCT/CN2023/118079
(87) International publication number: WO 2025/054781

(57) **Abstract**

Provided is a stem cell-derived embryo model construction method. The method comprises: culturing primordial stem cells *in vitro* or injecting primordial stem cells into an immunodeficient animal for differentiation, so as to obtain a stem cell-derived embryo model. By means of the method, during the preparation of the stem cell-derived embryo model, there is no need to add cytokines and small molecule pathway interference factors, and there is no need to perform gene editing and only a single starting cell type is used. The method has the advantages of simplicity and high repeatability, and is beneficial to large-scale preparation and application. Moreover, the prepared embryo-like model has all extraembryonic cell lineages and all embryonic cell lineages other than trophectoderm, can simulate the development process of a post-implantation embryo of Carnegie stage 3 to Carnegie stage 9 neurula, and then can be used for preparation of a variety of progenitor cell lineages, disease modeling, drug screening, and scientific researches.

## Description

### FIELD

The present disclosure relates to the field of biotechnology. Specifically, the present disclosure relates to a stem cell-derived embryo model and a construction method therefor and use thereof. More specifically, the present disclosure relates to a method for constructing a stem cell-derived embryo model *in vitro* or *in vivo* using stem cells.

### BACKGROUND

Human early embryonic development mainly proceeds through the stages of zygote development, morula, blastocyst, gastrulation, neurulation, and organogenesis. Due to ethical concerns and the limited availability of human tissues, many developmental stages, such as gastrulation, remain highly mysterious in the context of human development. Our current understanding of embryonic development largely derives from model organisms such as mice. However, substantial differences exist between mouse and human embryos at the early developmental stages. For instance, morphologically, after implantation, the mouse epiblast forms a cup-shaped structure, whereas the human embryo forms a bilaminar disc structure. Therefore, it is difficult to directly extrapolate research findings from other species to humans. In addition to model animals, human stem cell-based embryonic models are also important tools for studying early human embryonic development. Compared with human embryos, embryonic models derived from human stem cells for studying embryonic development also allow for gene editing.

Selecting an appropriate *in vitro* embryonic model for research can greatly help in understanding human embryonic development and the mechanisms of related diseases. However, current *in vitro* embryonic models, especially those simulating the gastrulation stage (such as gastruloids), contain only a limited number of cell types. As a result, these models can only partially recapitulate key developmental events and cannot faithfully reflect the full spectrum of embryonic processes.

Accordingly, it is essential to construct a model capable of efficiently simulating the complexity of embryogenesis.

### SUMMARY

The present disclosure aims to develop a scalable method for generating a three-dimensional cellular aggregate including both intraembryonic and extraembryonic tissues from a single starting cell type, without the need for cytokines or small-molecule pathway interference factors.

To this end, the present disclosure defines a three-dimensional (3D) cellular aggregate derived from the differentiation of human pluripotent stem cells or totipotent cells, the aggregate including both intraembryonic and extraembryonic tissues. The inventors have found that the embryoid tissue includes endoderm cells and their derivatives, mesoderm cells and their derivatives, and ectoderm cells and their derivatives; the extraembryonic tissue includes yolk sac endoderm cells and their derivatives, yolk sac mesothelial cells and their derivatives, extraembryonic mesoderm cells and their derivatives, and amniotic cells and their derivatives. The cell types of this model closely resemble those of the human embryos at the gastrulation stage, and therefore can serve as a model for studying diseases related to embryonic development.

Accordingly, in an aspect of the present disclosure, a method for constructing a stem cell-derived embryo model is provided. According to an embodiment of the present disclosure, the method includes: culturing naive stem cells *in vitro,* or injecting the naive stem cells into an immunodeficient animal for *in vivo* differentiation, to obtain the stem cell-derived embryo model. The inventors have found that, during the preparation of the stem cell-derived embryo model by this method, no cytokines, small-molecule pathway inhibitors or activators, or gene editing processes are required, and only a single starting cell type is used. The method has advantages of simplicity and high reproducibility, and is more favorable for large-scale application. In addition, the stem cell-derived embryo model thus prepared is a 3D embryo-like model with multi-lineage cell types, meaning that it possesses all extraembryonic and intraembryonic cell lineages except trophectoderm. The model is capable of simulating the entire developmental process from a post-implantation embryo at Carnegie stage 3 to a neurula at Carnegie stage 9, and thus can be used for the preparation of multiple progenitor cell lineages, disease modeling, drug screening, scientific researches, etc.

It should be noted that the term "naive pluripotent stem cells (naive PSCs)" as used in the present disclosure refers to a particular cellular state of pluripotent stem cells. In *in vitro* culture, pluripotent stem cells exist in at least two distinct states: naive and primed. Both states are capable of differentiating into the three germ layers of the embryo: endoderm, mesoderm, and ectoderm. According to an embodiment of the present disclosure, the starting cells used herein are naive-state stem cells (human naive cells). The naive cells according to an embodiment of the present disclosure most closely resemble the inner cell mass and possess the potential to differentiate into the epiblast (which later develops into the three germ layers) and the hypoblast (which later develops into a complete yolk sac).

According to an embodiment of the present disclosure, the method further includes at least one of the following additional technical features.

According to an embodiment of the present disclosure, the *in vitro* culture of naive stem cells includes: performing a first culture treatment on the naive stem cells in a first culture medium and a second culture medium to obtain a first cell population; performing a second culture treatment on the first cell population in a third culture medium to obtain a second cell population; and performing a third culture treatment on the second cell population in a fourth culture medium and a fifth culture medium to obtain the stem cell-derived embryo model. Different culture media used at various stages all require no addition of cytokines or small-molecule pathway interference factors. The cells are in a state of natural differentiation throughout the culture process. Naive stem cells can spontaneously differentiate into epiblast cells and hypoblast cells in the culture process without any artificial intervention or induction. Moreover, by using different culture media at different stages for culture and preparation, a stem cell-derived embryo model with a relatively complete range of extraembryonic lineages can be obtained, which can be used in simulation of embryonic development (especially human gastrulation), disease modeling, drug screening, scientific researches, etc.

According to an embodiment of the present disclosure, the naive stem cells are subjected to a pre-culture treatment in advance, and coverage of the naive stem cells subsequent to the pre-culture treatment ranges from 70% to 80% in a culture dish.

According to an embodiment of the present disclosure, a digestion treatment is performed on the naive stem cells subsequent to the pre-culture treatment.

According to an embodiment of the present disclosure, the digestion treatment is performed in the presence of TrypLE^{™} Express enzyme.

It should be noted that the "TrypLE^{™} Express enzyme" is a commercial enzyme, which can be used directly after purchase, or can be used after dilution with an EDTA solution. When diluting the TrypLE^{™} Express enzyme, a volume ratio of the TrypLE^{™} Express enzyme to the EDTA solution with a concentration of 0.3 mM to 0.7 mM ranges from 1: (0.8 to 1.2).

According to an embodiment of the present disclosure, the first culture medium includes a human primed pluripotent stem cell culture medium, penicillin-streptomycin, and a Rock pathway inhibitor.

It should be noted that the "human primed pluripotent stem cell culture medium" referred to in the present disclosure may be selected from mTeSR or other equivalent culture media, as long as it is capable of culturing human primed pluripotent stem cells.

According to an embodiment of the present disclosure, the second culture medium includes a human primed pluripotent stem cell culture medium and penicillin-streptomycin. Components of the culture medium are all basic components, requiring no addition of cytokines or small-molecule pathway interference factors. The cells are in a state of natural differentiation throughout the culture process. Naive stem cells can spontaneously differentiate into epiblast cells and hypoblast cells in the culture process without any artificial intervention or induction.

According to an embodiment of the present disclosure, a mass-volume fraction of the penicillin-streptomycin in the first culture medium or the second culture medium ranges from 0.5% to 1.5%.

According to an embodiment of the present disclosure, the Rock pathway inhibitor includes Y27632.

According to an embodiment of the present disclosure, a final concentration of Y27632 in the first culture medium ranges from 45 µM to 55 µM;

According to an embodiment of the present disclosure, the first culture treatment is performed in a low-adhesion 96-well round-bottom plate, a 3D scaffold, or a microfluidic device.

According to an embodiment of the present disclosure, the first culture treatment lasts for 5 days to 7 days.

According to a specific embodiment of the present disclosure, the first culture treatment lasts for 5 days.

According to an embodiment of the present disclosure, the first culture treatment includes a first-stage culture treatment and a second-stage culture treatment. The first-stage culture treatment is performed in the first culture medium. The second-stage culture treatment is performed in the second culture medium.

According to an embodiment of the present disclosure, the first-stage culture treatment lasts for 3 days.

According to an embodiment of the present disclosure, the first-stage culture treatment includes culturing the naive stem cells in the first culture medium for 1 day, replacing the first culture medium, and further culturing the naive stem cells in the first culture medium for 2 days.

According to an embodiment of the present disclosure, the second-stage culture treatment lasts for 2 days to 4 days.

According to an embodiment of the present disclosure, the second-stage culture treatment lasts for 2 days.

According to an embodiment of the present disclosure, the third culture medium includes a DMEM-F12 basal medium, an N2 supplement, glutamine, non-essential amino acids, heparin, and penicillin-streptomycin. Components of the culture medium are all basic components, requiring no addition of cytokines or small-molecule pathway interference factors. The cells are in a state of natural differentiation throughout the culture process. Primed stem cells can spontaneously and naturally differentiate into epiblast cells and hypoblast cells in the culture process without any artificial intervention or induction.

According to an embodiment of the present disclosure, a mass-volume fraction of the N2 supplement in the third culture medium ranges from 0.5% to 1.5%.

According to an embodiment of the present disclosure, a mass-volume fraction of the glutamine in the third culture medium ranges from 0.5% to 1.5%.

According to an embodiment of the present disclosure, a mass-volume fraction of the non-essential amino acids in the third culture medium ranges from 0.5% to 1.5%.

According to an embodiment of the present disclosure, a final concentration of the heparin in the third culture medium ranges from 0.5 µg/mL to 1.5 µg/mL.

According to an embodiment of the present disclosure, a mass-volume fraction of the penicillin-streptomycin in the third culture medium ranges from 0.5% to 1.5%.

According to an embodiment of the present disclosure, the second culture treatment lasts for 3 days to 5 days.

According to an embodiment of the present disclosure, prior to the third culture treatment, the second cell population is mixed with a matrix gel.

According to an embodiment of the present disclosure, a ratio of a volume of the matrix gel to the number of the second cell population is 30 µL of matrix gel: 1 second cell population.

It should be noted that the "second cell population" refers to a formed tissue aggregate, i.e., the stem cell-derived embryo model. Accordingly, a ratio of the volume of the matrix gel to the number of the stem cell-derived embryo model is 30 µL of matrix gel per one embryo-like model (EB/embryoid).

According to an embodiment of the present disclosure, the fourth culture medium includes a DMEM-F12 basal medium, a neural basal medium, an N2 supplement, glutamine, non-essential amino acids, insulin, β-mercaptoethanol, penicillin-streptomycin, and a vitamin A-free B27 supplement. The medium contains only nutritional supplements and is free of serum and cytokine. Therefore, the cells remain in a state of natural differentiation.

According to an embodiment of the present disclosure, the fifth culture medium includes a DMEM-F12 basal medium, a neural basal medium, an N2 supplement, glutamine, non-essential amino acids, insulin, β-mercaptoethanol, penicillin-streptomycin, and a vitamin A-containing B27 supplement. The medium contains only nutritional supplements and is free of serum and cytokine. Therefore, the cells remain in a state of natural differentiation.

According to an embodiment of the present disclosure, a volume ratio of the DMEM-F12 basal medium to the neural basal medium ranges from 1: (0.8 to 1.2).

According to an embodiment of the present disclosure, a mass-volume fraction of the N2 supplement in the fourth culture medium or the fifth culture medium ranges from 0.2% to 0.7%.

According to an embodiment of the present disclosure, a mass-volume fraction of the glutamine in the fourth culture medium or the fifth culture medium ranges from 0.5% to 1.5%.

According to an embodiment of the present disclosure, a mass-volume fraction of the non-essential amino acids in the fourth culture medium or the fifth culture medium ranges from 0.2% to 0.7%.

According to an embodiment of the present disclosure, a final concentration of the insulin in the fourth culture medium or the fifth culture medium ranges from 1 µg/mL to 1.5 µg/mL.

According to an embodiment of the present disclosure, a final concentration of the β-mercaptoethanol in the fourth culture medium or the fifth culture medium ranges from 0.1 µM to 0.25 µM.

According to an embodiment of the present disclosure, a mass-volume fraction of the penicillin-streptomycin in the fourth culture medium or the fifth culture medium ranges from 0.5% to 1.5%.

According to an embodiment of the present disclosure, a mass-volume fraction of the vitamin A-free B27 supplement in the fourth culture medium ranges from 0.2% to 0.7%.

According to an embodiment of the present disclosure, a mass-volume fraction of the vitamin A-containing B27 supplement in the fifth culture medium ranges from 0.2% to 0.7%.

According to an embodiment of the present disclosure, the third culture treatment includes a third-stage culture treatment and a fourth-stage culture treatment. The third-stage culture treatment is performed in the fourth culture medium. The fourth-stage culture treatment is performed in the fifth culture medium.

According to an embodiment of the present disclosure, the third-stage culture treatment lasts for 3 days to 5 days.

According to an embodiment of the present disclosure, the third-stage culture treatment lasts for 4 days.

According to an embodiment of the present disclosure, the fourth-stage culture treatment lasts for 13 days to 18 days.

According to an embodiment of the present disclosure, the fourth-stage culture treatment lasts for 16 days.

According to an embodiment of the present disclosure, the fourth-stage culture treatment is performed on a shaker.

According to an embodiment of the present disclosure, a rotational speed of the shaker ranges from 60 RPM to 80 RPM.

According to an embodiment of the present disclosure, prior to the injecting the naive stem cells into an immunodeficient animal for differentiation, the naive stem cells are mixed with a DMEM-F12 basal medium containing a matrix gel.

According to an embodiment of the present disclosure, a volume ratio of the DMEM-F12 basal medium to the matrix gel ranges from 1: (0.8 to 1.2).

According to an embodiment of the present disclosure, a ratio of the number of the naive stem cells to a volume of the DMEM-F12 basal medium containing the matrix gel ranges from 5⁴ cells: (0.8 to 1.2) µL.

According to an embodiment of the present disclosure, the immunodeficient animal is selected from, but is not limited to, an immunodeficient mouse, an immunodeficient rabbit, an immunodeficient cow, an immunodeficient pig, or an immunodeficient monkey.

According to an embodiment of the present disclosure, the naive stem cells are selected from, but are not limited to, at least one of pluripotent stem cells, totipotent stem cells, primitive endoderm cells, or trophectoderm cells.

According to an embodiment of the present disclosure, a source of the naive stem cells is selected from, but is not limited to, human, cow, monkey, pig, or mouse.

According to an embodiment of the present disclosure, the injecting is performed subcutaneously.

According to an embodiment of the present disclosure, the *in vivo* differentiation lasts for 13 days to 70 days.

According to an embodiment of the present disclosure, the naive stem cells are obtained by: performing a fourth culture treatment on the primed stem cells in a sixth culture medium to obtain a fourth cell population; performing a fifth culture treatment on the fourth cell population in a seventh culture medium to obtain a fifth cell population; performing a sixth culture treatment on the fifth cell population in an eighth culture medium to obtain the naïve stem cells. In some other embodiments, the naïve stem cells can also be induced using other culture media, such as 5iLAF culture medium, PXGL culture medium, HENSM culture medium, etc. The naive stem cells prepared by the method, when used in subsequent preparation of the stem cell-derived embryo model, require no addition of cytokines or small-molecule pathway interference factors and no gene editing, and can yield the stem cell-derived embryo model with a relatively complete range of extraembryonic lineages using only a single starting cell type.

According to an embodiment of the present disclosure, the sixth culture medium includes a human primed pluripotent stem cell culture medium and a Rock pathway inhibitor.

According to an embodiment of the present disclosure, the Rock pathway inhibitor includes Y27632.

According to an embodiment of the present disclosure, a final concentration of Y27632 in the sixth culture medium ranges from 5 µM to 15 µM.

According to an embodiment of the present disclosure, the fourth culture treatment lasts for 20 hours to 27 hours.

According to an embodiment of the present disclosure, each of the seventh culture medium and the eighth culture medium includes a neural basal medium, a DMEM-F12 basal medium, an N2 supplement, a B27 supplement, sodium pyruvate, non-essential amino acids, glutamine, penicillin-streptomycin, DZNep (a histone methyltransferase EZH2 inhibitor), TSA (a deacetylase inhibitor), an MEK inhibitor, a WNT signaling pathway inhibitor, a recombinant human leukemia inhibitory factor, an activin A, an L-ascorbic acid, and a matrix gel.

According to an embodiment of the present disclosure, a volume ratio of the neural basal medium to the DMEM-F12 basal medium is 1:1.

According to an embodiment of the present disclosure, a mass-volume fraction of the N2 supplement in the seventh culture medium or the eighth culture medium ranges from 0.5% to 1.5%.

According to an embodiment of the present disclosure, a mass-volume fraction of the B27 supplement in the seventh culture medium or the eighth culture medium ranges from 1% to 3%.

According to an embodiment of the present disclosure, a mass-volume fraction of the sodium pyruvate in the seventh culture medium or the eighth culture medium ranges from 0.5% to 1.5%.

According to an embodiment of the present disclosure, a mass-volume fraction of the non-essential amino acids in the seventh culture medium or the eighth culture medium ranges from 0.5% to 1.5%.

According to an embodiment of the present disclosure, a mass-volume fraction of the glutamine in the seventh culture medium or the eighth culture medium ranges from 0.5% to 1.5%.

According to an embodiment of the present disclosure, a mass-volume fraction of the penicillin-streptomycin in the seventh culture medium or the eighth culture medium ranges from 0.5% to 1.5%.

According to an embodiment of the present disclosure, a final concentration of the DZNep in the seventh culture medium ranges from 8 nM to 12 nM.

According to an embodiment of the present disclosure, a final concentration of the DZNep in the eighth culture medium ranges from 45 nM to 55 nM.

According to an embodiment of the present disclosure, a final concentration of the TSA in the seventh culture medium ranges from 4 nM to 6 nM.

According to an embodiment of the present disclosure, a final concentration of the TSA in the eighth culture medium ranges from 15 nM to 25 nM.

According to an embodiment of the present disclosure, the MEK inhibitor includes PD0325901.

According to an embodiment of the present disclosure, a final concentration of the PD0325901 in the seventh culture medium or the eighth culture medium ranges from 0.5 µM to 1.5 µM.

According to an embodiment of the present disclosure, the WNT signaling pathway inhibitor includes IWR-1.

According to an embodiment of the present disclosure, a final concentration of the IWR-1 in the seventh culture medium or the eighth culture medium ranges from 3 µM to 6 µM.

According to an embodiment of the present disclosure, a final concentration of the recombinant human leukemia inhibitory factor in the seventh culture medium or the eighth culture medium ranges from 15 ng/mL to 25 ng/mL.

According to an embodiment of the present disclosure, a final concentration of the activin A in the seventh culture medium or the eighth culture medium ranges from 15 ng/mL to 25 ng/mL.

According to an embodiment of the present disclosure, a final concentration of the L-ascorbic acid in the seventh culture medium or the eighth culture medium ranges from 45 µg/mL to 55 µg/mL.

According to an embodiment of the present disclosure, a volume ratio of the matrix gel in the seventh culture medium or the eighth culture medium ranges from 0.1% to 0.3%.

According to an embodiment of the present disclosure, the fifth culture treatment lasts for 12 hours to 24 hours.

According to an embodiment of the present disclosure, the sixth culture treatment lasts for 12 days.

In a second aspect of the present disclosure, a stem cell-derived embryo model is provided. According to an embodiment of the present disclosure, the stem cell-derived embryo model is constructed by the method described in the first aspect. The prepared embryo-like model has a relatively complete range of lineages and can be used in simulation of embryonic development (especially human gastrulation), disease modeling, drug screening, scientific researches, etc.

In a third aspect of the present disclosure, a stem cell-derived embryo model is provided. According to an embodiment of the present disclosure, the stem cell-derived embryo model includes an extraembryonic tissue and an intraembryonic tissue. Therefore, the stem cell-derived embryo model possesses all extraembryonic and intraembryonic cell lineages except trophectoderm. The model is capable of simulating the entire developmental process from a post-implantation embryo at Carnegie stage 3 to a neurula at Carnegie stage 9, and thus can be used for the preparation of multiple progenitor cell lineages, disease modeling, drug screening, scientific researches, etc.

According to an embodiment of the present disclosure, the stem cell-derived embryo model can further include at least one of the following additional technical features.

According to an embodiment of the present disclosure, the extraembryonic tissue includes: visceral endoderm, anterior visceral endoderm, yolk sac endoderm, extraembryonic mesoderm, mesothelial cells, allantois cells, mesoderm progenitor cells of hematopoietic precursor cells derived from extraembryonic mesoderm, hemangioblasts derived from extraembryonic mesoderm, endothelial cells derived from extraembryonic mesoderm, arterial endothelial cells derived from extraembryonic mesoderm, hematopoietic precursor cells derived from extraembryonic mesoderm, megakaryocytes derived from extraembryonic mesoderm, erythrocytes derived from extraembryonic mesoderm, myeloid precursor cells derived from extraembryonic mesoderm, amniotic ectoderm cells, and amniotic/non-neural ectoderm progenitor cells.

According to an embodiment of the present disclosure, the intraembryonic tissue includes epiblast, committed epiblast, primordial germ cell-like cells, definitive endoderm, primitive streak, emergent mesoderm, advanced mesoderm, neuroepithelium, and neural crest-derived cells.

According to an embodiment of the present disclosure, the stem cell-derived embryo model includes epiblast, committed epiblast, primordial germ cell-like cells, an endoderm lineage, a mesoderm lineage, and an ectoderm lineage.

According to an embodiment of the present disclosure, the endoderm lineage includes an intraembryonic tissue and an extraembryonic tissue. The intraembryonic tissue includes definitive endoderm. The extraembryonic tissue includes visceral endoderm, anterior visceral endoderm, and yolk sac endoderm.

According to an embodiment of the present disclosure, the mesoderm lineage includes an intraembryonic tissue and an extraembryonic tissue. The intraembryonic tissue includes primitive streak, emergent mesoderm, and advanced mesoderm. The extraembryonic tissue includes extraembryonic mesoderm, mesothelial cells, allantois cells, mesoderm progenitor cells of hematopoietic precursor cells derived from extraembryonic mesoderm, hemangioblasts, endothelial cells, arterial endothelial cells, hematopoietic precursor cells, megakaryocytes, erythrocytes, and myeloid precursor cells.

According to an embodiment of the present disclosure, the ectoderm lineage includes an intraembryonic tissue and an extraembryonic tissue. The intraembryonic tissue includes neuroepithelium and neural crest-derived cells. The extraembryonic tissue includes amniotic ectoderm cells and amniotic/non-neural ectoderm progenitor cells.

According to an embodiment of the present disclosure, genes expressed in the visceral endoderm include *HNF4A* and *SOX17*; genes expressed in the anterior visceral endoderm include *OTX2* and *CXCR4;* genes expressed in the yolk sac endoderm include *HNF4A,* and the yolk sac endoderm exhibits low or no expression of *SOX17*; genes expressed in the extraembryonic mesoderm cells include *ANXA1, LUM, NID2,* and *FRZB;* genes expressed in the mesothelial cells include *AQP1*; genes expressed in the allantois cells include *TBX4* and *HOXA13;* genes expressed in the mesoderm progenitor cells of hematopoietic precursor cells derived from the extraembryonic mesoderm include *BMP4* and *WNT5A;* genes expressed in the hemangioblasts derived from the extraembryonic mesoderm include *ETV2, TAL1, BMP4,* and *WNT5A;* genes expressed in the endothelial cells derived from the extraembryonic mesoderm include *CDH5* and *CD34;* genes expressed in the arterial endothelial cells derived from the extraembryonic mesoderm include *GJA4* and *GJA5*; genes expressed in the hematopoietic precursor cells derived from the extraembryonic mesoderm include *RUNX1* and *SPN;* genes expressed in the megakaryocytes derived from the extraembryonic mesoderm include *PPBP;* genes expressed in the erythrocytes derived from the extraembryonic mesoderm include *GATA1* and *HBE1;* genes expressed in the myeloid precursor cells derived from the extraembryonic mesoderm include *CSF1R*; genes expressed in the amniotic ectoderm cells include *ISL1* and *GABRP;* genes expressed in the amniotic/non-neural ectoderm progenitor cells include *CLDN10, AMR2, PCAT14,* and *VGLL1;* genes expressed in the epiblast include *POU5F1* and *DPPA4;* genes expressed in the committed epiblast include *TNFRSF12A* and *JUND;* genes expressed in the primordial germ cell-like cells include *NANOG, NANOS3, TFAP2C,* and *SOX17*; genes expressed in the definitive endoderm include *HHEX;* genes expressed in the primitive streak include *TBXT;* genes expressed in the emergent mesoderm include *LHX1*; genes expressed in the advanced mesoderm include *MYL7,* and the advanced mesoderm exhibits low or no expression of *TBXT;* genes expressed in the neuroepithelium include *PAX6;* and genes expressed in the neural crest-derived cells include *SOX10* and *MPZ.*

According to an embodiment of the present disclosure, the endoderm lineage expresses *FOXA2.* That is, the definitive endoderm, the visceral endoderm, the anterior visceral endoderm, and the yolk sac endoderm co-express *FOXA2.*

According to an embodiment of the present disclosure, genes expressed in an epiblast lineage include *POU5F1* and *DPPA4;* genes expressed in the committed epiblast include *TNFRSF12A* and *JUND;* genes expressed in the primordial germ cell-like cells include *NANOG, NANOS3, TFAP2C,* and *SOX17*; genes expressed in the endoderm lineage include *FOXA2;* genes expressed in the advanced mesoderm include *TBXT* and *MYL7;* genes expressed in the allantois cells include *TBX4* and *HOXA13;* genes expressed in the amniotic/non-neural ectoderm progenitor cells include *CLDN10, AMR2, PCAT14, and VGLL1;* genes expressed in the extraembryonic mesoderm include *ANXA1, LUM, NID2,* and *FRZB;* genes expressed in the definitive endoderm include *HHEX;* genes expressed in the visceral endoderm include *HNF4A* and *SOX17*; genes expressed in the anterior visceral endoderm include *OTX2* and *CXCR4;* genes expressed in the yolk sac endoderm include *HNF4A,* and the yolk sac endoderm exhibits low expression of *SOX17*; genes expressed in the primitive streak include *TBXT;* genes expressed in the emergent mesoderm include *LHX1*; genes expressed in the neuroepithelium include *PAX6*; genes expressed in the neural crest-derived cells include *SOX10* and *MPZ;* genes expressed in the amniotic ectoderm cells include *ISL1* and *GABRP;* genes expressed in the mesothelial cells include *AQP1*; genes expressed in the endothelial cells include *CDH5* and *CD34;* genes expressed in the arterial endothelial cells include *GJA4* and *GJA5*; genes expressed in the mesoderm progenitor cells of hematopoietic precursor cells include *BMP4* and *WNT5A;* genes expressed in the hemangioblasts include *ETV2, TAL1, BMP4, and WNT5A;* genes expressed in the hematopoietic precursor cells include *RUNX1* and *SPN;* genes expressed in the megakaryocytes include *PPBP;* genes expressed in the erythrocytes include *GATA1* and *HBE1*; and genes expressed in the myeloid precursor cells include *CSF1R.*

According to an embodiment of the present disclosure, a proportion of respective cell types in the extraembryonic tissue is: the visceral endoderm 0.46% to 11.57%, the anterior visceral endoderm 0.14% to 33.17%, the yolk sac endoderm 1.19% to 4.24%, the extraembryonic mesoderm 0.33% to 32.35%, the mesothelial cells 4.22% to 4.33%, the allantois cells 0.14% to 4.26%, the mesoderm progenitor cells of hematopoietic precursor cells derived from extraembryonic mesoderm 0.1% to 0.5%, the hemangioblasts 0.1% to 0.5%, the endothelial cells 1.40% to 4.79%, the arterial endothelial cells 0.04% to 0.1%, the hematopoietic precursor cells 0.01% to 0.1%, the megakaryocytes 0.01% to 0.1%, the erythrocytes 0.1% to 0.5%, the myeloid precursor cells 0.01% to 0.1%, the amniotic ectoderm cells 0.21% to 10.23%, and the amniotic/non-neural ectoderm progenitor cells 0.09% to 3.00%.

According to an embodiment of the present disclosure, a proportion of each major cell type in the intraembryonic tissue is: the epiblast 2.18% to 33.61%, the committed epiblast 2.57% to 2.86%, the primordial germ cell-like cells 0.02% to 0.19%, the definitive endoderm 0.48% to 1.63%, the primitive streak 0.31% to 0.90%, the emergent mesoderm 0.15% to 1.83%, the advanced mesoderm 1.61% to 12.81%, the neuroepithelium 0.18% to 3.13%, and the neural crest-derived cells 0.18% to 2.84%.

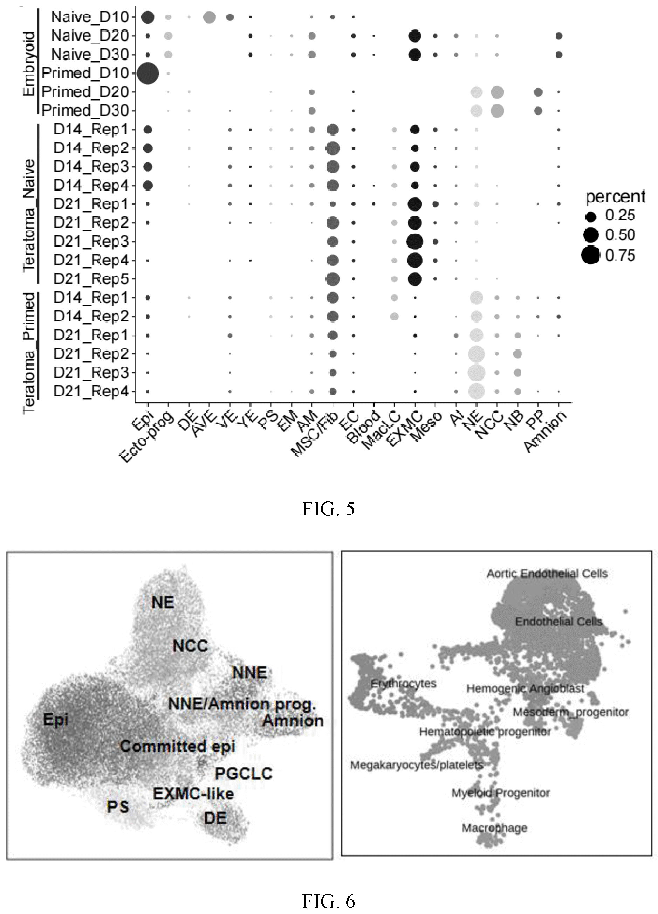

According to an embodiment of the present disclosure, proportions of respective cell types in the extraembryonic tissue and the intraembryonic tissue are as illustrated in FIG. 5.

According to an embodiment of the present disclosure, the stem cell-derived embryo model is capable of simulating cell types during a gastrulation stage.

In a fourth aspect of the present disclosure, use of the stem cell-derived embryo model described in the second aspect or the third aspect in constructing a Carnegie developmental stage model is provided.

According to an embodiment of the present disclosure, the Carnegie developmental stage is selected from developmental stage 3 to developmental stage 9.

In a fifth aspect of the present disclosure, use of the stem cell-derived embryo model described in the second aspect or the third aspect in drug screening is provided. The drug is used to intervene in embryonic development during the gastrulation stage.

In a sixth aspect of the present disclosure, use of the stem cell-derived embryo model described in the second aspect or the third aspect in constructing a disease model is provided. The disease model is selected from a developmental disease model.

According to an embodiment of the present disclosure, the developmental disease model is selected from an embryo implantation defect model or a Down syndrome model. Developmental abnormalities at this stage may lead to miscarriage, and defects in organ and brain development.

In a seventh aspect of the present disclosure, use of the stem cell-derived embryo model described in the second aspect or the third aspect in obtaining specific lineage progenitor cells is provided.

According to an embodiment of the present disclosure, the use includes expanding and culturing a specific lineage in the stem cell-derived embryo model to obtain the specific lineage progenitor cells.

According to an embodiment of the present disclosure, the use further includes performing gene editing or cell differentiation on the expanded and cultured specific lineage, or using the expanded and cultured specific lineage for cell therapy.

In an eighth aspect of the present disclosure, use of the stem cell-derived embryo model described in the second aspect or the third aspect in constructing a specific organoid model or a cellular 3D aggregate is provided.

According to an embodiment of the present disclosure, the use includes inducing the stem cell-derived embryo model into the specific organoid model or the cellular 3D aggregate using induction means.

According to an embodiment of the present disclosure, the induction means includes addition of a small molecule compound, co-culture with cells, or addition of a cytokine.

In a ninth aspect of the present disclosure, a method for screening a drug for intervening in embryonic development during a gastrulation stage is provided. According to an embodiment, the method includes: adding a candidate drug to a culture process of the method described in the first aspect for constructing the stem cell-derived embryo model, or bringing the candidate drug into contact with the stem cell-derived embryo model described in the second aspect or the third aspect; and determining, based on whether the stem cell-derived embryo model obtained through the method described in the first aspect or the stem cell-derived embryo model described in the second aspect or the third aspect undergoes an expected change, the candidate drug as a target drug.

According to an embodiment of the present disclosure, occurrence of the expected change in the stem cell-derived embryo model obtained by the method described in the first aspect after the adding the candidate drug, as compared with before the adding the candidate drug, indicates that the candidate drug is the target drug.

According to an embodiment of the present disclosure, occurrence of the expected change in the stem cell-derived embryo model described in the second aspect or the third aspect after the contact, as compared with before the contact, indicates that the candidate is the target drug.

### Advantageous effects:

The present disclosure has established the *in vivo* and *in vitro* methods for generating a three-dimensional aggregate (the stem cell-derived embryo model) including both the intraembryonic tissue and the extraembryonic tissue. Both the *in vivo* and *in vitro* methods require no cytokines or small-molecule pathway interference factors, involve no gene editing, and utilize only the single starting cell type. The methods are simple and highly reproducible, thus making them more suitable for large-scale applications. The stem cell-derived embryo model of the present disclosure includes both embryoid and extraembryonic tissues. Compared with currently available embryonic development models, the stem cell-derived embryo model of the present disclosure more closely resembles the *in vivo* state in terms of lineage diversity, which provides a better model for modeling embryonic development-related diseases.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and more understandable from the following description of embodiments taken in conjunction with the accompanying drawings.
FIG. 1 is a schematic diagram of a method for generating a stem cell-derived embryo model from pluripotent stem cells/totipotent stem cells *in vivo* and *in vitro* according to an embodiment of the present disclosure.
FIG. 2 is a bubble chart of single-cell data from experiments using an *in vivo* method and an *in vitro* method according to an embodiment of the present disclosure, showing expression levels of specific marker genes in different cell types, where a bubble size represents a proportion of gene expression in different cell types, and a bubble color represents a relative level of the gene expression.
FIG. 3 shows integrated single-cell UMAP plots of each of single-cell data from human embryos (6 days to 19 days), single-cell data from a stem cell-derived embryo model generated by 3D culture using an *in vitro* method according to an embodiment of the present disclosure, and single-cell data from a stem cell-derived embryo model formed by teratoma using an *in vivo* method according to an embodiment of the present disclosure.

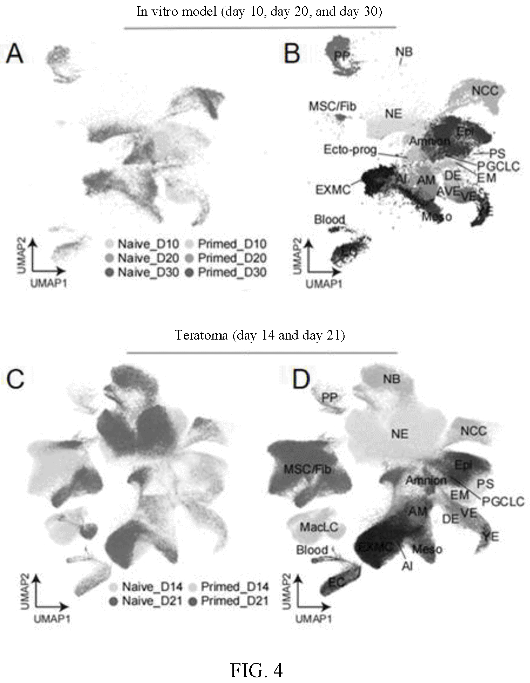

FIG. 4 shows a comparison of developmental potential of different stem cells differentiated using an *in vitro* method and an *in vivo* method according to an embodiment of the present disclosure, in which an upper part shows integrated single-cell UMAP plots of primed pluripotent stem cells and naive pluripotent stem cells after differentiation using the *in vitro* method according to an embodiment of the present disclosure, where A uses colors to label different sampling time points and B uses colors to label different cell types; a lower part shows integrated single-cell UMAP plots of primed pluripotent stem cells and naive pluripotent stem cells after differentiation using a teratoma method according to an embodiment of the present disclosure, where C uses colors to label different sampling time points and D uses colors to label different cell types.
FIG. 5 is a bubble chart of cell proportions after differentiation of different stem cells using an *in vitro* method and an *in vivo* method according to an embodiment of the present disclosure.
FIG. 6 shows a subgrouped UMAP plot of an epiblast lineage and its derived lineages (left) and a subgrouped UMAP plot of hematopoietic-related lineages (right) according to an embodiment of the present disclosure.
FIG. 7 shows identification of naive stem cells by real-time quantitative PCR and immunofluorescence staining.

### DETAILED DESCRIPTION

To facilitate the understanding of this technology, various terms and phrases are defined below. Additional definitions are set forth throughout the detailed description.

The term "stem cell" describes a cell capable of forming cells of many tissue types. Different types of stem cells exist. When a sperm fertilizes an egg, a single totipotent cell is formed, which has the ability to form an entire organism. Within the first hour after fertilization, this cell divides into identical totipotent cells. After approximately four days and several cycles of cell division, these totipotent stem cells begin to specialize. As totipotent cells become more specialized, they are then referred to as "pluripotent". Pluripotent cells can differentiate into every cell type in the body but cannot form the placenta or the supporting tissues necessary for fetal development. Since the differentiation potential of pluripotent stem cells is not "general", such cells are not called "totipotent", and they are not embryos. Pluripotent stem cells further specialize into multipotent stem cells, which are dedicated to differentiating into cells of specific lineages specialized for particular functions. Multipotent cells can differentiate into cell types contained in the tissue from which they originate. For example, blood stem cells can only differentiate into red blood cells, white blood cells, and platelets.

The term "embryonic structure" reflects any structure that occurs during development from fertilization to the fetus, including developmental stages of morula, blastocyst, and gastrula. Therefore, the term "embryonic structure" includes structures that have not yet cavitied into a blastocyst.

In the second week after fertilization, an inner cell mass proliferates and differentiates, gradually forming a disc-like bilaminar embryonic disc. Composed of two germ layers, it is called a bilaminar embryonic disc. The layer of columnar cells adjacent to the trophoblast is the epiblast, while the layer of cuboidal cells close to the blastocoel cavity side is the hypoblast. The inner and outer germ layer cells are closely attached to form the bilaminar embryonic disc, which is the primordium for embryonic body development. In mammalian embryology, as the cuboidal cells of the hypoblast gradually approach the blastocyst cells, the columnar cells of the epiblast are connected to trophoblast cells. The epiblast is also known as the primary ectoderm because it can develop into subsequent three germ layers: the ectoderm, mesoderm, and endoderm. These three germ layers are later associated with gastrulation development.

Under natural conditions, cell division of a fertilized egg produces a morula, which is a spherical embryonic cell structure containing 8 to 20 cells. Morula cells are totipotent, meaning that these cells can differentiate into any embryonic or extraembryonic cell type. As the morula matures, an internal cavity filled with fluid is formed. This event is associated with differentiation of morula cells into an outer layer (trophectoderm) and an inner cell mass (ICM or embryoblast). Such a cavitated cellular structure is called a blastocyst. Thus, the blastocyst is a stage in the development of a mammalian embryonic structure. The trophoblast cells of the blastocyst surround the inner cell mass (ICM) and a blastocoel cavity filled with fluid. The trophoblast cells of the blastocyst further develop into an ectoplacental cone, ultimately forming the placenta by binding to the uterine lining. The placenta is an organ that connects the developing fetus to the uterine wall, allowing nutrient uptake, waste elimination, and gas exchange via the maternal blood supply.

The ICM is composed of cells capable of differentiating into any body tissue, thus possessing pluripotency. Additionally, ICM cells form certain extraembryonic tissues, such as the yolk sac. The blastocyst further develops naturally through cell proliferation of the trophectoderm and the ICM and differentiation into cells other than embryonic stem cells. The first step in the development of ICM cells leads to the formation of the epiblast (which eventually forms tissues of an animal) and the hypoblast (which eventually forms tissues such as the yolk sac).

In the epiblast, cells continue to differentiate by forming three tissue layers (embryonic germ layers). These collectively form all the different tissue types present in an adult animal. The three germ layers of the embryo are the endoderm, ectoderm, and mesoderm. The endoderm develops into the gastrointestinal tract, respiratory tract, etc. The ectoderm develops into the nervous system, hair, nails, etc. The mesoderm develops into muscles, connective tissues, etc. Once the three embryonic germ layers are formed, the embryonic structure becomes a gastrula. Since cells in any embryonic germ layer cannot naturally differentiate into tissues derived from one of the other embryonic germ layers, cells in the embryonic germ layers are pluripotent, not totipotent or multipotent.

For the scope of the present disclosure, the term "embryo" is defined as any one in a series of developmental structures beginning from the blastocyst and including all subsequent developmental stages (including, for example, the gastrula) up to the formation of the fetus, including, for example, the embryonic developmental stage, the gastrula, up to the formation of the fetus. Thus, this term refers to those structures occurring in (natural) development from the blastocyst stage onward, and includes subsequent structures up to the fetal stage. The embryo becomes a fetus when all organs are basically formed.

A fetus is a mammal at a developmental stage after the embryonic period and before birth, whose organs are fully differentiated but not fully developed. Therefore, the terms "embryo" or "embryonic structure" do not encompass the fetus.

The term "Carnegie developmental stage" refers to a system proposed based on observations and classifications of human embryos, which is used to describe and compare characteristics and changes of human embryos at different developmental stages. The Carnegie developmental stages divide human embryonic development into 23 stages, named with Carnegie numbers from 1 to 23. Each stage represents a specific developmental stage and characteristics of the embryo, including aspects such as embryo size, morphological features, organ formation, and cell differentiation. These 23 stages are specifically divided as follows: stage 1 and stage 2: fertilized egg formation and cell division; stage 3: blastocyst expansion and implantation; stage 4 and stage 5: endoderm and ectoderm formation; stage 6 and stage 7: gastrula formation; stage 8 and stage 9: neurula formation; stage 10 and stage 11: organ primordia formation; stage 12 and stage 13: organ system formation; stage 14 and stage 15: appearance of fetal characteristics; stage 16 and stage 17: development of the nervous system and the muscular system; stage 18 and stage 19: refinement of organs and enhancement of functions; stage 20 to stage 23: final developmental stages of the embryo.

Examples of the present disclosure are described in detail below. The examples described below are exemplary and are intended only to explain the present disclosure, and should not be construed as limiting the present disclosure. Where specific techniques or conditions are not indicated in the examples, the procedures shall be carried out in accordance with the techniques or conditions described in the literature in the field or in accordance with the product specification. The reagents or instruments used without the indication of the manufacturers are all conventional products that can be purchased commercially.

### Example 1: Obtainment of naïve stem cells and early embryo-like totipotent stem cells

To obtain naive stem cells (naive PSCs), human primed PSCs (including commercial human pluripotent stem cell lines such as H9 or H1, or induced pluripotent stem cells) were washed once with DPBS. The human primed PSCs were dissociated into single cells using TrypLE^{™} Express: 0.5 mM EDTA (volume ratio: 1:1), and plated at a density ranging from 1,000 cells/cm² to 1,500 cells/cm² in a culture dish containing a human primed pluripotent stem cell culture medium supplemented with 10 µM Y-27632. After 24 hours, the culture medium was replaced with a 4CL culture medium. The 4CL culture medium contained neural basal medium (Gibco, 21103049) and DMEM/F12 basal medium (Gibco, 12634028), N2 supplement (Gibco, 17502048) at a mass-volume fraction ranging from 0.5% to 1.5%, B27 additive (Gibco, 17502048) at a mass-volume fraction ranging from 1% to 3%, sodium pyruvate (Corning, 25000CL) at a mass-volume fraction ranging from 0.5% to 1.5%, non-essential amino acids (Corning, 25025CL) at a mass-volume fraction ranging from 0.5% to 1.5%, glutamine (Gibco, 35050061) at a mass-volume fraction ranging from 0.5% to 1.5%, penicillin-streptomycin (HyClone, SV30010) at a mass-volume fraction ranging from 0.5% to 1.5%, 10 nM DZNep (Selleck, S7120), 5 nM TSA (Vetec, V900931), 1 µM PD0325901 (Axon, 1408), 5 µM IWR-1 (Sigma, I0161), 20 ng/mL recombinant human leukemia inhibitory factor (Peprotech, 300-05), 20 ng/mL activin A (Peprotech, 120-14E), 50 µg/ml L-ascorbic acid (Sigma, A8960), and 0.2% (v/v) Geltrex or Matrigel. The 4CL culture medium was replaced daily. The cells were passaged every 3 days to 4 days (1:5 to 1:8). After culture for approximately 12 days (3 passages), 4CL naive stem cells (naive PSCs) were obtained.

There are two methods for obtaining the early embryo-like totipotent stem cells. First method: the naïve stem cells were dissociated into single cells, and plated at a density ranging from 2,000 cells/cm² to 3,000 cells/cm² in a culture dish containing the 4CL culture medium supplemented with 5 µM Y-27632. After culture for 24 hours, the medium was replaced with an e4CL culture medium. The composition of the e4CL culture medium was identical to that of the 4CL culture medium, except that the concentration of DZNep was 50 nM and TSA was 20 nM. The culture lasted for 5 days. The e4CL culture medium was replaced daily. Second method: to directly produce the early embryo-like totipotent stem cells from the naive stem cells in the e4CL culture medium, human primed embryonic stem cells were dissociated into single cells, and plated at a density ranging from 2,000 cells/cm² to 3,000 cells/cm² in a culture dish containing the human primed pluripotent stem cell culture medium supplemented with 10 µM Y-27632 or TeSR-E8 culture medium. After culture for 24 hours, the medium was replaced with the e4CL culture medium. The e4CL culture medium was replaced daily. On day 4, the cells were digested into single cells for passaging, and continued to be cultured under e4CL conditions for another 4 days to obtain the early embryo-like totipotent stem cells.

### Example 2: Identification of 4CL naïve stem cells

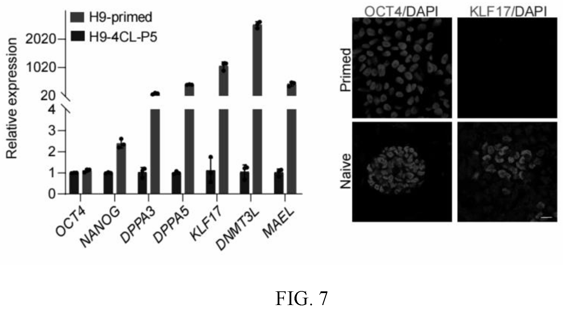

For the naive stem cells obtained in Example 1, expression levels of naive marker genes *DPPA3, DPPA5, KLF17, DNMT3L,* and *MAEL* were detected using the RT-qPCR experimental method. As illustrated in FIG. 7, compared with primed cells, the expression levels of *DPPA3, DPPA5, KLF17, DNMT3L,* and *MAEL* in the naive stem cells were significantly increased, and expression levels of pluripotency genes *OCT4* and *NANOG* remained substantially stable. In addition, high expression of the related protein *KLF17* was detected in the naive stem cells by immunofluorescence assay, confirming that the cells were in a naive state.

### Example 3: Generation of intraembryonic and extraembryonic tissues in an in vitro 3D environment

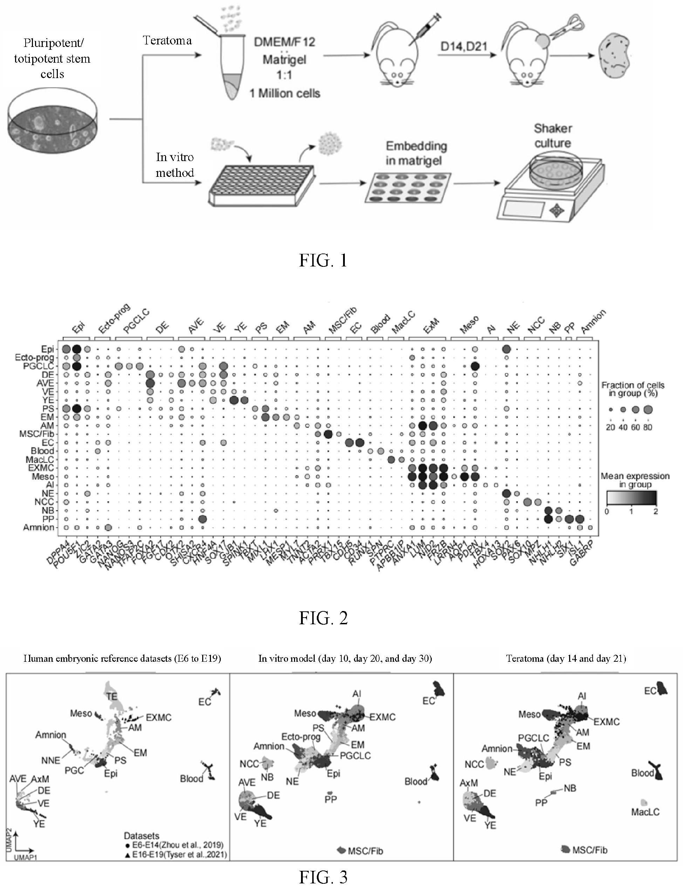

*In vitro* culture was performed in accordance with the method illustrated in FIG. 1. The naive stem cells or the early embryo-like totipotent stem cells obtained in Example 1 were cultured in the *in vitro* three-dimensional environment. A stem cell-derived embryo model was produced by a 3D organoid culture method. This method did not require cytokines, small-molecule pathway interference factors, or gene editing. Specifically, this method included preparing cells, forming cell spheres in a low-adhesion 96-well round-bottom plate using an embryoid body culture medium, inducing cell differentiation using an induction culture medium, and performing long-term culture in a long-term differentiation culture medium. Specific steps were as follows.

Day 0: When the confluency of cells (the naive stem cells or the early embryo-like totipotent stem cells obtained in Example 1) reached 70% to 80% of the area of a culture plate, clones were digested into single cells using Accutase enzyme (Gibco). 9,000 cells were resuspended in 150 µL of embryoid body culture medium (specific components of which are shown in Table 1) and seeded into a low-attachment 96-well culture plate.

**Table 1: Embryoid body culture medium**

| Embryoid body culture medium | |
|---|---|
| Component | Concentration |
| Human primed pluripotent stem cell culture medium (mTesR culture medium, STEMCELL Technologies) | |
| Penicillin-streptomycin (Hyclone) | 1% |
| Y27632 (Axon Medchem, 1681) | 50 µM |

Day 1: After 24 hours, small embryoid bodies with clear boundaries were observed under a tissue culture microscope. The embryoid bodies were then continuously cultured in a cell incubator at 37°C and 5% CO₂.

Day 2: The old embryoid body culture medium was removed without disturbing embryoid bodies at a bottom of the culture plate. 150 µL of fresh embryoid body culture medium was added to the culture plate.

Day 4: The fresh embryoid body culture medium without Y27632 (i.e., composed of mTesR and 1% penicillin-streptomycin) was added to the culture plate.

Day 6: The embryoid bodies were transferred to a low-attachment 24-well plate using a 200 µL wide-bore pipette tip. 500 µL of induction culture medium (specific components of which are shown in Table 2) was added.

**Table 2: Induction culture medium**

| Induction culture medium | |
|---|---|
| Component | Amount |
| DMEM-F12 basal medium (Invitrogen, cat. no. 11330-032 or 31330-038, depending on location) | 480 mL |
| N2 supplement (Invitrogen) | 5 mL |
| Glutamine TM (Invitrogen, cat. no. 35050-038) | 5 mL |
| Non-essential amino acids (Sigma, cat. no. M7145) | 5 mL |
| Heparin (Sigma, cat. no. H3149) | 500 µg |
| Penicillin-streptomycin (Hyclone) | 5 mL |

Day 8: The old induction culture medium was removed. 500 µL of fresh induction culture medium was added to the culture plate.

Day 10: The embryoid bodies had formed spheres and become tissue masses. The tissue masses were transferred into a matrix gel Matrigel.
1) The matrix gel Matrigel was dissolved at 4°C for 30 minutes.
2) A sealing film was placed over a tip-loading area of a 200 µL pipette tip, covering an area of (4×4) 16 wells. The sealing film was pressed with a finger to create small depressions. Sterilization under UV light lasted for 30 minutes.
3) The tissue masses were transferred into each pre-prepared depression using the 200 µL wide-bore pipette tip.
4) The excess culture medium was carefully removed from each depression using a 200 µL pipette tip.
5) Approximately 30 µL of growth factor-reduced matrix gel Matrigel (BD Biosciences, 356230) was immediately added to the tissue mass in each depression to fill the depression.
6) The tissue mass was moved as close to a center of the matrix gel Matrigel droplet as possible using a 10 µL pipette tip.
7) The sealing film setup was placed in a 60 mm² culture dish and incubated at 37°C for 20 minutes to 30 minutes to allow the matrix gel droplet to encapsulate the tissue masses.
8) 5 mL of long-term differentiation culture medium supplemented with a vitamin A-free B27 supplement was added to the 60 mm² culture dish (specific components of which are shown in Table 3).
9) The sealing film was moved using a sterile forceps. The culture dish was shaken to detach the matrix gel Matrigel droplet from the sealing film. If any droplets remain on the sealing film, one end of the sealing film can be fixed with the forceps, and the sealing film can be shaken in the culture medium to detach the remaining droplets. The detached matrix gel Matrigel droplets were placed in a cell culture incubator for continued culture.

Day 12: 5 mL of long-term differentiation culture medium supplemented with the vitamin A-free B27 supplement was added to the culture dish.

Day 14: 5 mL of long-term differentiation culture medium supplemented with a vitamin A-containing B27 supplement was added to the culture dish (specific components of which are shown in Table 3). The culture dish was transferred to an incubator equipped with a shaker set at 70 rpm. The culture medium was replaced every 3 days to 4 days.

**Table 3: Long-term differentiation culture medium**

| Long-Term differentiation culture medium | |
|---|---|
| Component | Amount |
| DMEM-F12 (Invitrogen, cat. no. 11330-032 or 31330-038, depending on location) | 250 mL |
| Neural basal medium (Neurobasal^{™} Medium, Thermo Fisher) | 250 mL |
| N2 supplement (Invitrogen) | 2.5 mL |
| Glutamine TM (Invitrogen, cat. no. 35050-038) | 5 mL |
| Non-essential amino acids (Sigma, cat. no. M7145) | 2.5 mL |
| Insulin (Sigma, cat. no. I9278-5ML) | 125 µL |
| β-mercaptoethanol (Merck, cat. no. 8057400005) | 1.75 µL |
| Penicillin-streptomycin (Hyclone) | 5 mL |
| Vitamin A-free B27 supplement (Invitrogen, cat. no. 12587010)/vitamin A-containing B27 supplement (Invitrogen, cat. no. 17504044) | 5 mL |

### Example 4: In vivo generation of intraembryonic and extraembryonic tissues

*In vivo* culture was performed in accordance with the method illustrated in FIG. 1. Cell aggregates were produced by transplanting cells into an immunodeficient mouse to form a teratoma. This method includes mixing the naive stem cells or the early embryo-like totipotent stem cells obtained in Example 1 with a matrix gel, and injecting the mixture into the mouse to form the cell aggregates having a 3D structure. Specific operational steps are as follows.

### 1) Animal preparation

Male NOD-scid-IL2Rg-/- mice (WuXi AppTec) were bred and maintained in an SPF facility until 4 weeks to 6 weeks of age.

### 2) Cell preparation

One million naive stem cells or early embryo-like totipotent stem cells obtained in Example 1 were separated from the culture medium. They were then collected and resuspended in 200 µL of pre-cooled mixture with a volume ratio of 1:1 of DMEM/F12 basal medium (Invitrogen, cat. no. 11330-032 or 31330-038) and a matrix gel, to obtain a resuspension. Before transplantation, the resuspension was kept on ice. Transplantation should be carried out immediately.

### 3) Transplantation experiment

The resuspension from step 2) was collected into a 1 mL syringe and administered via subcutaneous injection to male NOD-scid-IL2Rg-/- mice aged 4 weeks to 6 weeks.

To avoid stress reactions, mice should be prepared at least one week in advance. For the teratoma formation experiment, 200 µl of pre-cooled mixture with a volume ratio of 1:1 of DMEM/F12 basal medium and Matrigel was used. One million cells were contained in a 1 mL sterile syringe. The abdomen of the mouse was exposed to facilitate injection. The needle of the sterile syringe was inserted obliquely through the skin of the abdominal of the mouse. The cell suspension was injected subcutaneously into male NOD-scid-IL2Rg-/- mice aged 4 weeks to 6 weeks. The needle of the sterile syringe was slowly withdrawn to prevent the cell suspension from flowing out of the injection site.

### 4) Animal monitoring during teratoma formation

After injection, the mice were bred and maintained in an SPF-grade facility for 2 weeks to 3 weeks. The size of the teratoma growing at the injection site was observed visually. Typically, the teratoma was harvested 2 weeks to 3 weeks after injection.

### 5) Collection of human cells from the teratoma

2 weeks to 3 weeks after injection, the mice were euthanized, and the teratoma was harvested. The collected teratoma was used for frozen sectioning, cell separation, single-cell transcriptome analysis, or other purposes.

### Example 5: Identification of intraembryonic and extraembryonic cell types generated by in vivo and in vitro differentiation methods via single-cell sequencing

To systematically analyze the entire cell lineages in a naïve stem cell-derived embryo-like model and teratoma model, single-cell RNA sequencing (scRNA-seq) analysis was performed on the embryo model (day 10, day 20, and day 30) and the teratoma (day 14 and day 21) using the DNBelab C4 droplet platform. After quality control, 75,326 single cells from the embryo model and 397,684 single cells from the teratoma were obtained for further analysis. As illustrated in FIG. 2, uniform manifold approximation and projection (UMAP) analysis was used to identify embryo-like cell types, including epiblast (Epi, *POU5F1+* and *DPPA4+*) and committed epiblast (*TNFRSF12A+*/*JUND+*), specifically including primordial germ cell-like cells (PGCLC, *NANOG+*/*NANOS3+*/*TFAP2C+*/*SOX17+*); endoderm lineage *(FOXA2+*) including definitive endoderm (DE, *HHEX+*), visceral endoderm (VE, *HNF4A+*/*SOX17+*), anterior visceral endoderm (AVE, *OTX2+*/*CXCR4+*), yolk sac endoderm (YE, *HNF4A+*/*SOX17-*); mesoderm lineage including primitive streak (PS, *TBXT+*), emergent mesoderm (EM, *LHX1+*); advanced mesoderm cells (AM, *TBXT-*/*MYL7+*), allantois cells (Al, *TBX4+*/*HOXA13+*), amniotic/non-neural ectoderm progenitor cells (*CLDN10, AMR2+*/*PCAT14+*/*VGLL1+*), and extraembryonic mesoderm cells (EXMC) expressing mesoderm characteristics (*ANXA1, LUM, NID2*/*FRZB*) but not expressing markers of any embryonic counterparts; mesoderm-derived mesothelial cells (*AQP1+*), endothelial cells (*CDH5+*/*CD34+*), arterial endothelial cells (*GJA4+*/*GJA5+*), mesoderm progenitor cells of hematopoietic precursor cells (*BMP4+*/*WNT5A+*), hemangioblasts (*ETV2+*/*TAL1+*/*BMP4+*/*WNT5A+*), hematopoietic precursor cells (*RUNX1+*/*SPN+*), megakaryocytes (*PPBP*), erythrocytes (*GATA1+*/*HBE1+*), and myeloid precursor cells (*CSF1R+*); ectoderm lineage including neuroepithelium (NE, *PAX6+*), neural crest-derived cells (NCC, *SOX10+*/*MPZ+*), and amniotic ectoderm cells (*ISL1+*/*GABRP+*)*.* Through further subclustering, as illustrated in FIG. 6, the UMAP analysis also identified cell types such as committed epiblast (*TNFRSF12A+*/*JUND+*), arterial endothelial cells (*GJA4+*/*GJA5+*), mesoderm progenitor cells of hematopoietic precursor cells (*BMP4+*/*WNT5A+*), hemangioblasts (*ETV2+*/*TAL1+*/*BMP4+*/*WNT5A+*), hematopoietic precursor cells (*RUNX1+*/*SPN+*), megakaryocytes (*PPBP*), erythrocytes (*GATA1+*/*HBE1+*), and myeloid precursor cells (*CSF1R+*)*.*

To evaluate and analyze the developmental progression of the naive stem cell-derived embryo model and teratoma, scRNA-seq data were integrated with human embryonic reference datasets, including human blastocysts at the pre-gastrulation stage (E6 to E14), and Carnegie stage 7 (CS7; E16 to E19) human embryos during gastrulation. As illustrated in FIG. 3, the UMAP not only demonstrated an excellent match of cell type characteristics for both embryonic and extraembryonic lineages, but also indicated that the cell type differentiation follows the same process as development. Notably, the model of the present disclosure includes neuroectodermal cells and all other cell types found during CS7 gastrulation, suggesting that the model of the present disclosure has progressed beyond the gastrulation stage into the early organogenesis state. The identity of the neuroectodermal lineage was further validated by integrating a monkey embryo dataset (CS7 to CS11). Neither the teratoma nor the embryo model contained the trophectoderm lineage (which was later developed into the placenta). This limitation means that the model of the present disclosure cannot form a complete embryo.

### Example 6: Comparison of differentiation potential of different embryonic stem cells under in vitro 3D culture methods

Cell preparation: Human primed PSCs were maintained in the mTeSR culture medium (STEMCELL Technologies). When the human primed PSCs reached confluency ranging from 70% to 80%, the culture medium was removed during passaging or *in vitro* 3D culture experiment. 1 mL PBS was added along the wall of the culture dish. The cells were gently washed to remove the residual culture medium. The PBS was removed. 1 mL TrypLE^{™} Express: 0.5 mM EDTA (volume ratio: 1:1) digestion solution was added, placed in a 37°C incubator for about 5 minutes of digestion, and observed under a microscope. When cell edges became clear after digestion, a pipette was used to add 1 mL of fresh medium and the cells were pipetted. The cell suspension was transferred to a 1.5 mL EP tube. Centrifugation was performed at 300 g for 3 minutes. The supernatant was removed. The cells were resuspended with an appropriate amount of fresh culture medium. Cell count was conducted using a counting chamber or a cell counter. 9,000 cells were resuspended in 150 µL of embryoid body medium (specific components of which are shown in Table 1) and seeded into a low-attachment 96-well culture plate. Subsequent specific steps for *in vitro* 3D culture are described in Example 3.

To compare differentiation potential between primed stem cells and naive stem cells under the *in vitro* method, primed and naive stem cell-derived 3D products cultured to specific time points were digested into single cells using TrypLE^{™} Express: 0.5 mM EDTA (volume ratio: 1:1). Single-cell RNA sequencing (scRNA-seq) library construction and analysis were performed on these 3D differentiation products (day 10, day 20, and day 30) using the DNBelab C4 droplet platform (ref). As illustrated in FIG. 4 and FIG. 5, the differentiation products derived from the primed stem cells and the naïve stem cells were mainly composed of embryonic cell types, specifically epiblast cell types, whereas extraembryonic cell types such as anterior visceral endoderm and visceral endoderm derived from the hypoblast were only present in the differentiation products derived from the naive stem cells and completely absent in the differentiation products derived from the primed stem cells. Further, FIG. 5 reveals that, the proportion of respective cell types in the extraembryonic tissue from the naive stem cells was: the visceral endoderm 0.46% to 11.57%, the anterior visceral endoderm 0.14% to 33.17%, the yolk sac endoderm 1.19% to 4.24%, the extraembryonic mesoderm 0.33% to 32.35%, the mesothelial cells 4.22% to 4.33%, the allantois cells 0.14% to 4.26%, the mesoderm progenitor cells of hematopoietic precursor cells derived from extraembryonic mesoderm 0.1% to 0.5%, the hemangioblasts 0.1% to 0.5%, the endothelial cells 1.40% to 4.79%, the arterial endothelial cells 0.04% to 0.1%, the hematopoietic precursor cells 0.01% to 0.1%, the megakaryocytes 0.01% to 0.1%, the erythrocytes 0.1% to 0.5%, the myeloid precursor cells 0.01% to 0.1%, the amniotic ectoderm cells 0.21% to 10.23%, and the amniotic/non-neural ectoderm progenitor cells 0.09% to 3.00%; and the proportion of each major cell type in the intraembryonic tissue was: the epiblast 2.18% to 33.61%, the committed epiblast 2.57% to 2.86%, the primordial germ cell-like cells 0.02% to 0.19%, the definitive endoderm 0.48% to 1.63%, the primitive streak 0.31% to 0.90%, the emergent mesoderm 0.15% to 1.83%, the advanced mesoderm 1.61% to 12.81%, the neuroepithelium 0.18% to 3.13%, and the neural crest-derived cells 0.18% to 2.84%.

The unique differentiation ability of the naive stem cells mimicked the characteristics of an inner cell mass of the blastocyst, i.e., the ability to generate both epiblast and hypoblast lineages. Moreover, on day 20 and day 30, the differentiation products derived from the naive stem cells contained more lineages, including extraembryonic lineages and primordial germ cell-like cell types, whereas the differentiation products derived from the primed stem cells were primarily composed of neuroectodermal cell types. Thus, it is evident that, under the *in vitro* 3D culture method, the naive stem cells exhibit superior differentiation potential compared with the primed stem cells.

### Example 7: Comparison of differentiation potential of different embryonic stem cells in teratoma formation

When the human primed PSCs reached confluency ranging from 70% to 80%, 1 mL PBS was added. The cells were gently washed to remove the residual culture medium. The PBS was removed. 1 mL TrypLE^{™} Express: 0.5 mM EDTA (volume ratio: 1:1) was added.

The cells were digested into single cells. One million human primed PSCs were resuspended in 100 µL of pre-cooled mixture with a volume ratio of 1:1 of DMEM/F12 basal medium (Invitrogen, cat. no. 11330-032 or 31330-038) and the matrix gel Matrigel^{®}, to obtain a resuspension. Before transplantation, the resuspension was kept on ice. Transplantation should be carried out immediately. Subsequent transplantation experiments and specific experimental steps are described in Example 4.

To compare differentiation potential between the primed stem cells and the naive stem cells under the teratoma formation method, single-cell RNA sequencing (scRNA-seq) library construction and analysis were performed on the obtained teratoma (day 14 and day 21). As illustrated in FIG. 4 and FIG. 5, the lineages of the teratoma derived from the primed stem cells were comparable to the lineages of the *in vitro* 3D cultured differentiation products derived from the naive stem cells. Compared with the lineages of the teratoma derived from the naive stem cells, the lineages of the teratoma derived from the primed stem cells mainly contained neuroectodermal cell types and a small number of extraembryonic lineages, whereas the teratoma derived from the naive stem cells had more extraembryonic lineages and primordial germ cell-like cell types. Further, FIG. 5 also reveals that, the proportion of respective cell types in the extraembryonic tissue in the teratoma derived from the naive stem cells was: the visceral endoderm 0.46% to 11.57%, the anterior visceral endoderm 0.14% to 33.17%, the yolk sac endoderm 1.19% to 4.24%, the extraembryonic mesoderm 0.33% to 32.35%, the mesothelial cells 4.22% to 4.33%, the allantois cells 0.14% to 4.26%, the mesoderm progenitor cells of hematopoietic precursor cells derived from extraembryonic mesoderm 0.1% to 0.5%, the hemangioblasts 0.1% to 0.5%, the endothelial cells 1.40% to 4.79%, the arterial endothelial cells 0.04% to 0.1%, the hematopoietic precursor cells 0.01% to 0.1%, the megakaryocytes 0.01% to 0.1%, the erythrocytes 0.1% to 0.5%, the myeloid precursor cells 0.01% to 0.1%, the amniotic ectoderm cells 0.21% to 10.23%, and the amniotic/non-neural ectoderm progenitor cells 0.09% to 3.00%; and the proportion of each major cell type in the intraembryonic tissue was: the epiblast 2.18% to 33.61%, the committed epiblast 2.57% to 2.86%, the primordial germ cell-like cells 0.02% to 0.19%, the definitive endoderm 0.48% to 1.63%, the primitive streak 0.31% to 0.90%, the emergent mesoderm 0.15% to 1.83%, the advanced mesoderm 1.61% to 12.81%, the neuroepithelium 0.18% to 3.13%, and the neural crest-derived cells 0.18% to 2.84%.

Thus, it is evident that, under the teratoma formation method, the naive stem cells exhibit superior differentiation potential compared with the primed stem cells.

Throughout this specification, description with reference to "an embodiment," "some embodiments," "an example," "a specific example," or "some examples," means that a particular feature, structure, material, or characteristic described in connection with the embodiment or example is included in at least one embodiment or example of the present disclosure. The appearances of the above phrases in various places throughout this specification are not necessarily referring to the same embodiment or example of the present disclosure. Further, the particular features, structures, materials, or characteristics described here may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that the above embodiments are merely exemplary, and cannot be construed to limit the present disclosure. For those skilled in the art, changes, alternatives, and modifications can be made to the embodiments without departing from the scope of the present disclosure.

## Claims

1. A method for constructing a stem cell-derived embryo model, the method comprising:
culturing naive stem cells *in vitro,* or injecting the naive stem cells into an immunodeficient animal for *in vivo* differentiation, to obtain the stem cell-derived embryo model.

2. The method according to claim 1, wherein said culturing naive stem cells *in vitro* comprises:
performing a first culture treatment on the naive stem cells in a first culture medium and a second culture medium to obtain a first cell population;
performing a second culture treatment on the first cell population in a third culture medium to obtain a second cell population; and
performing a third culture treatment on the second cell population in a fourth culture medium and a fifth culture medium to obtain the stem cell-derived embryo model.

3. The method according to claim 2, wherein the naive stem cells are subjected to a pre-culture treatment in advance, and coverage of the naive stem cells subsequent to the pre-culture treatment ranges from 70% to 80% in a culture dish;
optionally, a digestion treatment is performed on the naive stem cells subsequent to the pre-culture treatment; and
optionally, the digestion treatment is performed in the presence of TrypLE^{™} Express enzyme.

4. The method according to claim 2, wherein the first culture medium comprises a human primed pluripotent stem cell culture medium, penicillin-streptomycin, and a Rock pathway inhibitor;
optionally, the second culture medium comprises a human primed pluripotent stem cell culture medium and penicillin-streptomycin;
optionally, a mass-volume fraction of the penicillin-streptomycin in the first culture medium or the second culture medium ranges from 0.5% to 1.5%;
optionally, the Rock pathway inhibitor comprises Y27632;
optionally, a final concentration of Y27632 in the first culture medium ranges from 45 µM to 55 µM;
optionally, the first culture treatment is performed in a low-adhesion 96-well round-bottom plate, a 3D scaffold, or a microfluidic device;
optionally, the first culture treatment lasts for 5 days to 7 days, preferably 5 days; optionally, the first culture treatment comprises a first-stage culture treatment in the first culture medium and a second-stage culture treatment in the second culture medium;
optionally, the first-stage culture treatment lasts for 3 days, and optionally, the first-stage culture treatment comprises: culturing the naive stem cells in the first culture medium for 1 day, replacing the first culture medium, and further culturing the naive stem cells in the first culture medium for 2 days; and
optionally, the second-stage culture treatment lasts for 2 days to 4 days, and preferably 2 days.

5. The method according to claim 2, wherein the third culture medium comprises a DMEM-F12 basal medium, an N2 supplement, glutamine, non-essential amino acids, heparin, and penicillin-streptomycin;
optionally, a mass-volume fraction of the N2 supplement in the third culture medium ranges from 0.5% to 1.5%;
optionally, a mass-volume fraction of the glutamine in the third culture medium ranges from 0.5% to 1.5%;
optionally, a mass-volume fraction of the non-essential amino acids in the third culture medium ranges from 0.5% to 1.5%;
optionally, a final concentration of the heparin in the third culture medium ranges from 0.5 µg/mL to 1.5 µg/mL;
optionally, a mass-volume fraction of the penicillin-streptomycin in the third culture medium ranges from 0.5% to 1.5%; and
optionally, the second culture treatment lasts for 3 days to 5 days.

6. The method according to claim 2, wherein, prior to the third culture treatment, the second cell population is mixed with a matrix gel.

7. The method according to any one of claims 2 to 6, wherein the fourth culture medium comprises a DMEM-F12 basal medium, a neural basal medium, an N2 supplement, glutamine, non-essential amino acids, insulin, β-mercaptoethanol, penicillin-streptomycin, and a vitamin A-free B27 supplement;
optionally, the fifth culture medium comprises a DMEM-F12 basal medium, a neural basal medium, an N2 supplement, glutamine, non-essential amino acids, insulin, β-mercaptoethanol, penicillin-streptomycin, and a vitamin A-containing B27 supplement;
optionally, a volume ratio of the DMEM-F12 basal medium to the neural basal medium ranges from 1: (0.8 to 1.2);
optionally, a mass-volume fraction of the N2 supplement in the fourth culture medium or the fifth culture medium ranges from 0.2% to 0.7%;
optionally, a mass-volume fraction of the glutamine in the fourth culture medium or the fifth culture medium ranges from 0.5% to 1.5%;
optionally, a mass-volume fraction of the non-essential amino acids in the fourth culture medium or the fifth culture medium ranges from 0.2% to 0.7%;
optionally, a final concentration of the insulin in the fourth culture medium or the fifth culture medium ranges from 1 µg/mL to 1.5 µg/mL;
optionally, a final concentration of the β-mercaptoethanol in the fourth culture medium or the fifth culture medium ranges from 0.1 µM to 0.25 µM;
optionally, a mass-volume fraction of the penicillin-streptomycin in the fourth culture medium or the fifth culture medium ranges from 0.5% to 1.5%;
optionally, a mass-volume fraction of the vitamin A-free B27 supplement in the fourth culture medium ranges from 0.2% to 0.7%;
optionally, a mass-volume fraction of the vitamin A-containing B27 supplement in the fifth culture medium ranges from 0.2% to 0.7%;
optionally, the third culture treatment comprises a third-stage culture treatment in the fourth culture medium and a fourth-stage culture treatment in the fifth culture medium;
optionally, the third-stage culture treatment lasts for 3 days to 5 days, and preferably 4 days;
optionally, the fourth-stage culture treatment lasts for 13 days to 18 days, and preferably 16 days;
optionally, the fourth-stage culture treatment is performed on a shaker; and
optionally, a rotational speed of the shaker ranges from 60 RPM to 80 RPM.

8. The method according to claim 1, wherein, prior to said injecting the naive stem cells into an immunodeficient animal for differentiation, the naive stem cells are mixed with a DMEM-F12 basal medium containing a matrix gel;
optionally, a volume ratio of the DMEM-F12 basal medium to the matrix gel ranges from 1: (0.8 to 1.2); and
optionally, a ratio of the number of the naive stem cells to a volume of the DMEM-F12 basal medium containing the matrix gel ranges from 5⁴ cells: (0.8 to 1.2) µL.

9. The method according to claim 1 or 8, wherein the immunodeficient animal is selected from an immunodeficient mouse, an immunodeficient rabbit, an immunodeficient cow, an immunodeficient pig, or an immunodeficient monkey;
optionally, the naive stem cells are selected from at least one of pluripotent stem cells, totipotent stem cells, primitive endoderm cells, or trophectoderm cells;
optionally, a source of the naive stem cells is selected from human, cow, monkey, pig, or mouse;
optionally, said injecting is performed subcutaneously; and
optionally, the *in vivo* differentiation lasts for 13 days to 70 days.

10. A stem cell-derived embryo model, constructed by the method according to any one of claims 1 to 9.

11. A stem cell-derived embryo model, comprising an extraembryonic tissue and an intraembryonic tissue.

12. The stem cell-derived embryo model according to claim 11, wherein the extraembryonic tissue comprises: visceral endoderm, anterior visceral endoderm, yolk sac endoderm, extraembryonic mesoderm, mesothelial cells, allantois cells, mesoderm progenitor cells of hematopoietic precursor cells from extraembryonic mesoderm, hemangioblasts from extraembryonic mesoderm, endothelial cells from extraembryonic mesoderm, arterial endothelial cells from extraembryonic mesoderm, hematopoietic precursor cells from extraembryonic mesoderm, megakaryocytes from extraembryonic mesoderm, erythrocytes from extraembryonic mesoderm, myeloid precursor cells from extraembryonic mesoderm, amniotic ectoderm cells, and amniotic/non-neural ectoderm progenitor cells;
optionally, the intraembryonic tissue comprises epiblast, committed epiblast, primordial germ cell-like cells, definitive endoderm, primitive streak, emergent mesoderm, advanced mesoderm, neuroepithelium, and neural crest-derived cells.

13. The stem cell-derived embryo model according to claim 12, wherein genes expressed in the visceral endoderm comprise *HNF4A* and *SOX17*; genes expressed in the anterior visceral endoderm comprise *OTX2* and *CXCR4;* genes expressed in the yolk sac endoderm comprise *HNF4A,* and the yolk sac endoderm exhibits low or no expression of *SOX17*; genes expressed in the extraembryonic mesoderm cells comprise *ANXA1, LUM, NID2,* and *FRZB;* genes expressed in the mesothelial cells comprise *AQP1*; genes expressed in the allantois cells comprise *TBX4* and *HOXA13*; genes expressed in the mesoderm progenitor cells of hematopoietic precursor cells from the extraembryonic mesoderm comprise *BMP4* and *WNT5A;* genes expressed in the hemangioblasts from the extraembryonic mesoderm comprise *ETV2, TAL1, BMP4, and WNT5A;* genes expressed in the endothelial cells from the extraembryonic mesoderm comprise *CDH5* and *CD34;* genes expressed in the arterial endothelial cells from the extraembryonic mesoderm comprise *GJA4* and *GJA5*; genes expressed in the hematopoietic precursor cells from the extraembryonic mesoderm comprise *RUNX1* and *SPN;* genes expressed in the megakaryocytes from the extraembryonic mesoderm comprise *PPBP;* genes expressed in the erythrocytes from the extraembryonic mesoderm comprise *GATA1* and *HBE1;* genes expressed in the myeloid precursor cells from the extraembryonic mesoderm comprise *CSF1R*; genes expressed in the amniotic ectoderm cells comprise *ISL1* and *GABRP;* genes expressed in the amniotic/non-neural ectoderm progenitor cells comprise *CLDN10, AMR2, PCAT14,* and *VGLL1*; genes expressed in the epiblast comprise *POU5F1* and *DPPA4;* genes expressed in the committed epiblast comprise *TNFRSF12A* and *JUND;* genes expressed in the primordial germ cell-like cells comprise *NANOG, NANOS3, TFAP2C,* and *SOX17;* genes expressed in the definitive endoderm comprise *HHEX;* genes expressed in the primitive streak comprise *TBXT;* genes expressed in the emergent mesoderm comprise *LHX1;* genes expressed in the advanced mesoderm comprise *MYL7,* and the advanced mesoderm exhibits low or no expression of *TBXT;* genes expressed in the neuroepithelium comprise *PAX6*; and genes expressed in the neural crest-derived cells comprise *SOX10* and *MPZ;*
optionally, the definitive endoderm, the visceral endoderm, the anterior visceral endoderm, and the yolk sac endoderm co-express *FOXA2.*

14. The stem cell-derived embryo model according to claim 12, wherein a proportion of respective cell types in the extraembryonic tissue is: the visceral endoderm 0.46% to 11.57%, the anterior visceral endoderm 0.14% to 33.17%, the yolk sac endoderm 1.19% to 4.24%, the extraembryonic mesoderm 0.33% to 32.35%, the mesothelial cells 4.22% to 4.33%, the allantois cells 0.14% to 4.26%, the mesoderm progenitor cells of hematopoietic precursor cells from extraembryonic mesoderm 0.1% to 0.5%, the hemangioblasts 0.1% to 0.5%, the endothelial cells 1.40% to 4.79%, the arterial endothelial cells 0.04% to 0.1%, the hematopoietic precursor cells 0.01% to 0.1%, the megakaryocytes 0.01% to 0.1%, the erythrocytes 0.1% to 0.5%, the myeloid precursor cells 0.01% to 0.1%, the amniotic ectoderm cells 0.21% to 10.23%, and the amniotic/non-neural ectoderm progenitor cells 0.09% to 3.00%;
optionally, a proportion of respective major cell types in the intraembryonic tissue is: the epiblast 2.18% to 33.61%, the committed epiblast 2.57% to 2.86%, the primordial germ cell-like cells 0.02% to 0.19%, the definitive endoderm 0.48% to 1.63%, the primitive streak 0.31% to 0.90%, the emergent mesoderm 0.15% to 1.83%, the advanced mesoderm 1.61% to 12.81%, the neuroepithelium 0.18% to 3.13%, and the neural crest-derived cells 0.18% to 2.84%.

15. The stem cell-derived embryo model according to any one of claims 11 to 14, wherein the proportions of respective cell types in the extraembryonic tissue and the intraembryonic tissue are as represented in FIG. 5.

16. The stem cell-derived embryo model according to any one of claims 10 to 15, wherein the stem cell-derived embryo model is capable of simulating cell types during a gastrulation stage.

17. Use of the stem cell-derived embryo model according to any one of claims 10 to 16 in the construction of a Carnegie developmental stage model,
optionally, the Carnegie developmental stage is selected from developmental stage 3 to developmental stage 9.

18. Use of the stem cell-derived embryo model according to any one of claims 10 to 16 in drug screening, wherein the drug is used to intervene in embryonic development during the gastrulation stage.

19. Use of the stem cell-derived embryo model according to any one of claims 10 to 16 in the construction of a disease model, wherein the disease model comprises a developmental disease model,
optionally, the developmental disease model comprises an embryo implantation defect model or a Down syndrome model.

20. Use of the stem cell-derived embryo model according to any one of claims 10 to 16 in obtaining specific lineage progenitor cells.

21. The use according to claim 20, comprising culturing and expanding a specific lineage in the stem cell-derived embryo model to obtain the specific lineage progenitor cells,
optionally, further comprising performing gene editing or cell differentiation on the specific lineage subjected to said culturing and expanding, or using the specific lineage subjected to said culturing and expanding for cell therapy.

22. Use of the stem cell-derived embryo model according to any one of claims 10 to 16 in the construction of a specific organoid model or a cellular 3D aggregate.

23. The use according to claim 22, comprising inducing the stem cell-derived embryo model into the specific organoid model or the cellular 3D aggregate using induction means,
optionally, the induction means comprises addition of a small molecule compound, co-culture with cells, or addition of a cytokine.

24. A method for screening a drug for intervening in embryonic development during a gastrulation stage, the method comprising:
adding a candidate drug to a culture process of the method for constructing the stem cell-derived embryo model according to any one of claims 1 to 9, or bringing a candidate drug into contact with the stem cell-derived embryo model according to any one of claims 10 to 16; and
determining, when the stem cell-derived embryo model obtained by the method according to any one of claims 1 to 9 or the stem cell-derived embryo model according to any one of claims 10 to 16 undergoes an expected change, the candidate drug as a target drug.

25. The method according to claim 24, wherein an indicator that the candidate drug is the target drug is that the stem cell-derived embryo model obtained by the method according to any one of claims 1 to 9 undergoes the expected change subsequent to said adding the candidate drug, as compared with prior to said adding the candidate drug.

26. The method according to claim 24, wherein an indicator that the candidate drug is the target drug is that the stem cell-derived embryo model according to any one of claims 10 to 16 undergoes the expected change subsequent to the contact, as compared with prior to the contact.
